# EUROPÄISCHE PATENTSCHRIFT

(11) **EP 3 534 983 B1**
(45) Veröffentlichungstag und Bekanntmachung des Hinweises auf die Patenterteilung: **10.03.2021**
(21) Anmeldenummer: 17793838.8
(22) Anmeldetag: 27.10.2017
(51) Int. Cl.: A61M 1/16, A61L 2/10, A61L 2/24, E05B 1/00

(54) **MEDIZINISCHES GERÄT**
MEDICAL DEVICE
APPAREIL MÉDICAL

(30) Priorität: 02.11.2016 DE 102016013087
(43) Veröffentlichungstag der Anmeldung: 11.09.2019
(73) Patentinhaber: Fresenius Medical Care Deutschland GmbH, 61352 Bad Homburg (DE)
(72) Erfinder: BREHM, Winfried, 97461 Hofheim (DE)
(74) Vertreter: Herrmann, Uwe
(86) Internationale Anmeldenummer: PCT/EP2017/001265
(87) Internationale Veröffentlichungsnummer: WO 2018/082807

(56) Entgegenhaltungen:
- EP-A2- 0 448 122
- WO-A1-2011/012717
- US-A1- 2005 267 233
- US-A1- 2009 117 001
- US-A1- 2014 137 369
- US-A1- 2014 208 541
- US-A1- 2016 000 951

## Beschreibung

Die vorliegende Erfindung betrifft ein medinzisches Gerät mit einer Griffanordnung, insbesondere mit einer Griffanordnung für eine Tür des medizinischen Gerätes, wobei es sich bei dem medinzischen Gerät vorzugsweise um ein Blutbehandlungsgerät und besonders bevorzugt um ein Dialysegerät handelt.

Ein selbstreinigender Türgriff ist beispielsweise aus der Druckschrift US2014/208541 bekannt. Die Druckschrift US 2014/0137369 A1 offenbart einen ähnlichen Türgriff. Auch die Druckschrift WO2011/012717A1 befasst sich mit der Verbesserung der Hygiene bei Türgriffen. Gemäß der EP0448122A2 ist zur Verbesserung der Hygiene bei Türgriffen eine Desinfektionseinrichtung vorgesehen. Weiterhin offenbart die Druckschrift US2009/0117001A1 ein System, welches bei bestimmten Events eine Desinfektion mittls UV-Licht ausführt.

Insbesondere im medizinischen Bereich ist die Sicherstellung der Keimfreiheit von großer Bedeutung. So ist es bekannt, dass sich insbesondere an Türgriffen eine Vielzahl von Keimen sammeln kann, mit denen ein den Türgriff betätigender Nutzer in Kontakt kommt, was möglicherweise nachteilige gesundheitliche Konsequenzen haben kann. Daher besteht die Notwendigkeit der Desinfektion, wozu heute beispielsweise Desinfektionslösungen verwendet werden, die z.B. als Spray appliziert werden.

Aus dem Stand der Technik ist es weiterhin bekannt, eine Oberflächendesinfektion z.B. durch UV-Licht vorzunehmen. So ist es bekannt, dass beispielsweise UV-C oder UV-A Licht desinfizierende Wirkungen entfalten und daher zur Desinfektion geeignet sind.

Unabhängig von der Tatsache, dass bereits hinreichend Möglichkeiten und Mittel zur Desinfektion zur Verfügung stehen, ist es jedoch im Alltag kaum möglich, beispielsweise einen Türgriff nach jeder Berührung zu desinfizieren, so dass bei einer erneuten Berührung stets ein gewisses Risiko einer Infektion bzw. Kontamination besteht.

Der vorliegenden Erfindung liegt daher die Aufgabe zugrunde, ein medinzisches Gerät der eingangs genannten Art dahingehend weiterzubilden, dass sichergestellt werden kann, dass die Oberfläche des Griffes desinfiziert ist.

Diese Aufgabe wird durch ein medinzisches Gerät mit den Merkmalen des Anspruchs 1 gelöst. Danach ist vorgesehen, dass die Griffanordnung wenigstens einen Griff aufweist, der ringförmig ausgebildet ist und dass die Griffanordnung des Weiteren wenigstens eine mit Licht, vorzugsweise mit UV-Licht, arbeitende Desinfektionseinheit aufweist, die angeordnet ist, den Griff zu desinfizieren, wobei die Griffanordnung des Weiteren wenigstens eine Antriebseinheit aufweist, die ausgebildet ist, den Griff an der Desinfektionseinheit entlang zu bewegen, so dass der Griff von der Desinfektionseinheit mit Licht beaufschlagt wird.

Der vorliegenden Erfindung liegt somit der Gedanke zugrunde, den Griff der Griffanordnung, d.h. den durch die Hand berührten Teil zum Öffnen einer Tür, Klappe, Lade, Fenster etc. des medizinischen Gerätes entlang einer oder mehrerer Desinfektionseinheiten zu bewegen, wozu zumindest eine Antriebseinheit vorgesehen ist, die diese Drehbewegung des Griffes ausführt.

Der ringförmige Griff wird somit entlang der Desinfektionseinheit bewegt, womit sichergestellt ist, dass dessen Oberfläche desinfiziert wird.

Diese Bewegung kann manuell durch einen Nutzer initiiert werden, wozu wenigstens ein Betätigungselement, wie z.B. ein Schalter oder Berührungssensor vorgesehen ist, mit dem die Griffanordnung in Verbindung steht oder das einen Bestandteil der Griffanordnung bildet, wobei das Betätigungselement so ausgebildet ist, dass es die Antriebseinheit nach der Betätigung durch einen Nutzer aktiviert. Der Nutzer betätigt somit das Betätigungselement, was zur Folge hat, dass der ringförmige Griff entlang der Desinfektionseinheit bewegt wird und dabei an seiner Oberfläche desinfiziert wird.

Erfindungsgemäß ist vorgesehen, dass wenigstens ein Sensor, insbesondere ein Berührungssensor vorgesehen ist, mit dem die Griffanordnung in Verbindung steht oder der einen Bestandteil der Griffanordnung bildet, wobei der Sensor ausgebildet ist, die Berührung oder Bewegung des Griffs und/oder eines durch die Griffanordnung zu bewegenden Elementes zu erfassen und bei oder nach einer derartigen Erfassung die Antriebseinheit zu aktivieren.

In diesem Fall ist wenigstens eine Steuereinheit oder dergleichen vorgesehen, die mit dem Sensor in Verbindung steht, so dass diese das Sensorsignal erfasst und daraufhin (gleichzeitig oder mit einem zeitlichen Versatz) die Antriebseinheit in Gang setzt. Diese Ausführung hat den Vorteil, der der Nutzer nicht an die Aktivierung des Desinfektionsvorgangs denken muss, sondern dass dieser automatisiert abläuft.

Der oder die Sensoren können an oder in der Griffanordnung angeordnet sein oder auch an dem Element, wie z.B. an einer Tür oder Fenster, an der sich die Griffanordnung befindet. So ist es beispielsweise auch möglich, zu erfassen, dass sich eine Tür geöffnet hat, wie z.B. mittels eines Näherungssensors, und dann die Antriebseinheit zu aktivieren.

Vorzugsweise ist der Sensor so angeordnet, dass er eine Berührung oder Bewegung des Griffs erfasst und sodann die Antriebseinheit in Gang setzt.

Die Desinfektionseinheit ist gemäß einer bevorzugten Ausgestaltung der Erfindung nur dann in Betrieb, wenn auch die Antriebseinheit in Betrieb ist.

Erfindungsgemäß werden die Antriebsmittel erst mit einem zeitlichen Versatz zur Erkennung der Griffbenutzung aktiviert. So kann der Nutzer den Griff z.B. zum Öffnen einer Tür betätigen und anschließend kann die Antriebseinheit gestartet werden.

IErfindungsgemäß ist der Sensor elektrisch leitend und insbesondere über wenigstens einen Schleifkontakt mit dem Griff verbunden. Der Griff besteht vorzugsweise insgesamt oder wenigstens teilweise aus einem elektrisch leitenden Material.

Um eine vollständige Desinfektion zu ermöglichen, kann die Antriebseinheit derart ausgebildet sein, dass diese den Griff solange bewegt, so dass jeder Umfangsabschnitt des Griffes zumindest einmal an der Desinfektionseinheit entlang bewegt wird, d.h. der Ring zumindest einmal rotiert wird. Denkbar ist auch, den Griff nur so weit zu drehen, dass der vor Beginn der Drehung außerhalb des Gehäuses der Griffanordnung liegende Bereich desinfiziert wird.

Um den Griff nicht nur z.B. an seiner Außen- oder Innenseite, sondern über den gesamten Umfang in Querschnittsrichtung zu desinfizieren, kann vorgesehen sein, dass die Desinfektionseinheit den Griff teilweise oder vollständig umgreift und vorzugsweise ihrerseits ringförmig ausgebildet ist. Auch ist es denkbar, in Querschnittsrichtung mehrere Desinfektionseinheiten vorzusehen, so dass dieses Ziel erreicht wird.

Vorzugsweise ist die wenigstens eine Desinfektionseinheit so ausgebildet, dass der Griff nach der Desinfektion auf seiner Oberfläche vollständig desinfiziert ist.

Um eine geordnete Bewegung des Griffes zu erreichen, kann vorgesehen sein, dass eine oder mehrere Führungselemente, insbesondere Führungsrollen vorhanden sind, die den Griff bei seiner Bewegung führen.

Dabei kann wenigstens eines der Führungselemente angetrieben sein und somit gleichzeitig die Antriebseinheit oder einen Teil der Antriebseinheit bilden.

Denkbar ist es, dass die Antriebseinheit derart ausgebildet ist, dass der Griff in einer Richtung bewegt wird, in der er zunächst an dem oder den Führungselementen entlang bewegt wird und anschließend an der Desinfektionseinheit.

Die Desinfektionseinheit ist vorzugsweise derart ausgebildet, UV-C oder UV-A Licht abzugeben. Jedoch sind von der Erfindung auch andere Wellenlängenbereiche erfasst, wie z.B. der untere Wellenlängenbereich des sichtbaren Lichtes, z.B. ein Wellenlängenbereich zwischen 350 nm und 450 nm.

Die wenigstens eine Desinfektionseinheit kann eine oder mehrere UV-LEDs aufweisen.

Gemäß einer weiteren Ausgestaltung der Erfindung ist der Griff innen hohl ausgeführt, was den Vorteil mit sich bringt, dass der Griff vergleichsweise leicht ist und daher mit geringem Kraftaufwand bewegt werden kann.

Weiterhin kann vorgesehen sein, dass der Griff insgesamt oder wenigstens an seiner Oberfläche aus Kunststoff oder aus Metall, vorzugsweise aus Edelstahl besteht.

Weiterhin ist es denkbar, dass der Griff eine Oberfläche oder eine Oberflächenbeschichtung aufweist, die katalytisch oder photokatalytisch wirkende Stoffe enthält, insbesondere Titandioxid. In diesem Fall wird eine weitere Verbesserung der desinfizierenden Wirksamkeit erreicht. Es können dabei auch oder alternativ andere Lichtquellen als UV-Lichtquellen eingesetzt werden, um eine desinfizierende Wirkung zu entfalten, z.B. Lichtquellen, die Licht mit einer Wellenlänge von 380 nm abgeben.

Die vorliegende Erfindung betrifft des Weiteren die Verwendung einer Griffanordnung gemäß dem kennzeichnenden Teil eines der Ansprüche 1 bis 13 als Türgriff für ein medizinisches Gerät, insbesondere für ein Blutbehandlungsgerät, wie z.B. ein Dialysegerät.

Weitere Einzelheiten und Vorteile der Erfindung werden anhand eines in der Zeichnung dargestellten Ausführungsbeispiels näher erläutert.

Es zeigen:
- Figur 1:: eine perspektivische Ansicht auf die Griffanordnung mit geschlossenem und mit offenem Gehäuse,
- Figur 2:: eine schematische Ansicht der Griffanordnung in der Draufsicht in einer weiteren Ausführungsform und
- Figur 3:: eine perspektivische Ansicht einer Tür mit einer Griffanordnung gemäß der Erfindung.

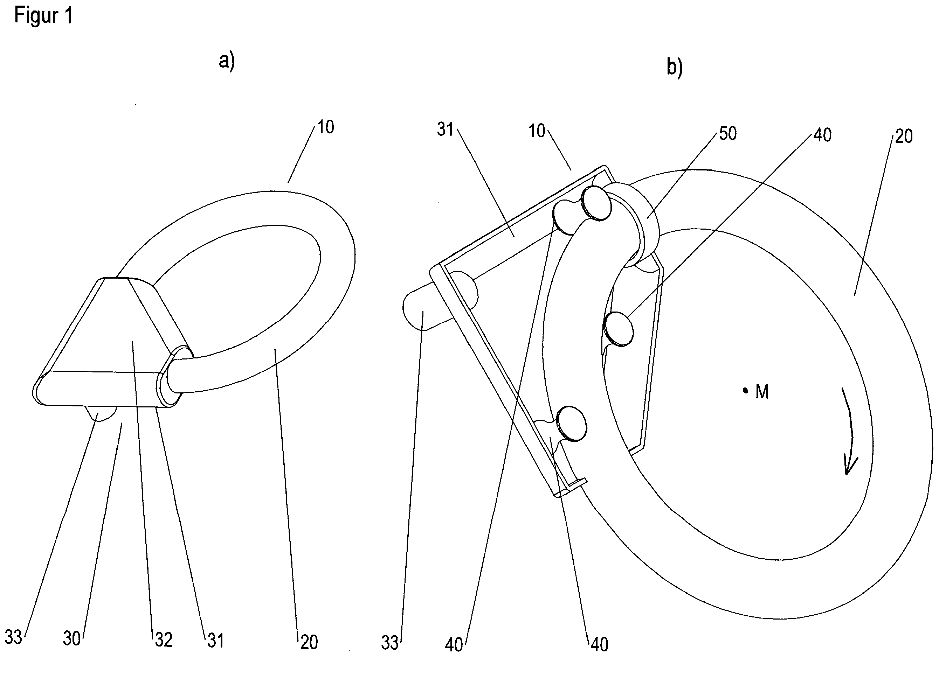

Figur 1 zeigt mit dem Bezugszeichen 10 die Griffanordnung als Ganze.

Die Griffanordnung umfasst einen ringförmigen Griff 20, der das eigentliche durch den Nutzer zu ergreifende Griffteil bildet und der beispielsweise innen hohl ausgeführt sein kann und der z.B. aus Edelstahl oder auch aus einem anderen elektrisch leitfähigen Material bzw. aus einem anderen Metall besteht.

Die Griffanordnung umfasst des Weiteren ein Gehäuse 30, das aus zwei Halbschalen besteht, die z.B. miteinander verrastet werden können.

Figur 1a zeigt die obere 32 sowie die untere Halbschale 31, d.h. das geschlossene Gehäuse, und Figur 1b zeigt nur die untere Halbschale 31. An der unteren Halbschale befindet sich ein Befestigungselement 33 zur Fixierung der Griffanordnung 10 an einer Tür oder dergleichen.

Das Gehäuse weist eine Einlassöffnung und eine Auslassöffnung auf, deren Größe in etwa der des Außendurchmessers des Griffes entspricht und durch die der sich bewegende Griff in das Gehäuse eintritt und aus diesem austritt.

Wie dies aus Figur 1b hervorgeht, befinden sich in dem Gehäuse 30 Führungsrollen 40, die den ringförmigen Griff 20 entlang einer Bahn führen, die der Gestalt des Ringes entspricht.

In dem hier dargestellten Ausführungsbeispiel sind drei Führungsrollen 40 vorgesehen, jedoch können auch mehr oder weniger als drei Führungsrollen vorhanden sein.

Grundsätzlich sind auch anderer Führungselemente, wie z.B. Ringe, Stege oder dergleichen denkbar, die den Griff auf seiner Bahn führen.

Eine, mehrere oder alle Führungsrollen 40 sind mit einem (nicht dargestellten) Elektromotor verbunden oder weisen einen Elektromotor auf, der geeignet ist, die Führungsrollen in eine Rotationsbewegung um deren Längsachse zu versetzen. Durch den Kontakt der Führungsrollen 40 mit dem Griff 20 wird dieser in Pfeilrichtung in eine Drehbewegung versetzt, d.h. der Griff dreht sich um einen Punkt bzw. Mittelpunkt M, wobei er durch die Führungsrollen 40 geführt ist.

Bei einem in der Draufsicht kreisrunden Griff befindet sich der Mittelpunkt M, d.h. die Drehachse genau im Kreismittelpunkt. Von der Erfindung sind auch andere Formen als kreisrunde Griffe umfasst, wie beispielsweise elliptische Ausführungen.

Vorzugsweise steht die Drehachse, um die der Griff gedreht wird, senkrecht oder im Winkel zur Senkrechten auf der durch den Griff gebildeten Ebene.

Das Bezugszeichen 50 kennzeichnet eine UV-Lichtquelle, die ebenfalls ringförmig ausgebildet ist und den Griff 20 in seiner Querschnittsrichtung vollständig umgreift. Die UV-Lichtquelle 50 ist so ausgebildet, dass der Ring in Querschnittsrichtung vollumfänglich mit UV-Licht (z.B.UV-C Licht) beaufschlagt wird.

Wie dies aus Figur 1b hervorgeht, durchläuft der Griff 20 zunächst den Bereich der Führungsrollen 40 und gelangt dann durch die UV-Lichtquelle 50. Der so desinfizierte Griffabschnitt verlässt sodann wieder das Gehäuse 30.

In dem Gehäuse 30 ist ein nicht dargestellter Sensor vorhanden, der mit dem Griff 20 elektrisch leitend z.B. über einen Schleifkontakt oder eine Rolle verbunden ist. Es ist eine ebenfalls nicht dargestellte Steuereinheit vorgesehen, die mit dem Sensor und der Antriebseinheit in Verbindung steht und die nach jeder Berührung oder Betätigung des Griffs 20 im Gehäuse einen Motor aktiviert, der den Ring bzw. den Griff 20 dreht.

Die Steuereinheit kann innerhalb oder außerhalb des Gehäuses angeordnet sein.

Als Sensor kommt beispielsweise ein kapazitiver Berührungssensor in Betracht.

Grundsätzlich ist ein Sensor im Rahmen der vorliegenden Erfindung jedes beliebige Element, mittels dessen eine Berührung oder Bestätigung des Griffs oder des Teils, wie z.B. der Tür erfasst werden kann, an dem sich die Griffanordnung befindet.

Nach der Aktivierung durch die Steuereinheit wird der Ring bzw. Griff 20 zumindest oder genau so weit gedreht, bis der vorher offen liegende (d.h. nicht im Gehäuse befindliche) Bereich desinfiziert ist. Damit ist sichergestellt, dass der zuletzt berührte Bereich desinfiziert wird. Beim Drehen des Griffs 20 wird dessen Oberfläche mittels einer oder mehrerer UV-LEDs bestrahlt und dabei desinfiziert.

Vorzugsweise ist die Bestrahlung so angeordnet, dass diese nach dem Passieren der Führungselemente erfolgt, wie dies aus Figur 1b ersichtlich ist; so wird eine Rückkontamination vermieden.

Der Griff weist keine Ecken und Kanten auf und durch seine Form wird eine vollständige Desinfektion seiner Oberfläche gewährleistet. Zudem bietet der ringförmige Griff eine angenehme und bequeme Betätigung.

Ein Öffnen einer Tür ist mit wenig Kontakt, beispielsweise nur mit einem Finger, möglich und ein Abrutschen vom Griff ist nahezu ausgeschlossen. Die Ringform bietet außerdem den Vorteil, dass eine einfach ausgebildete und der Größe des Rings entsprechende Halterung für dessen Befestigung an der Tür oder dergleichen verwendet werden kann. Die Bestrahlungseinheit kann auf einfache Art und Weise in die Halterung integriert werden.

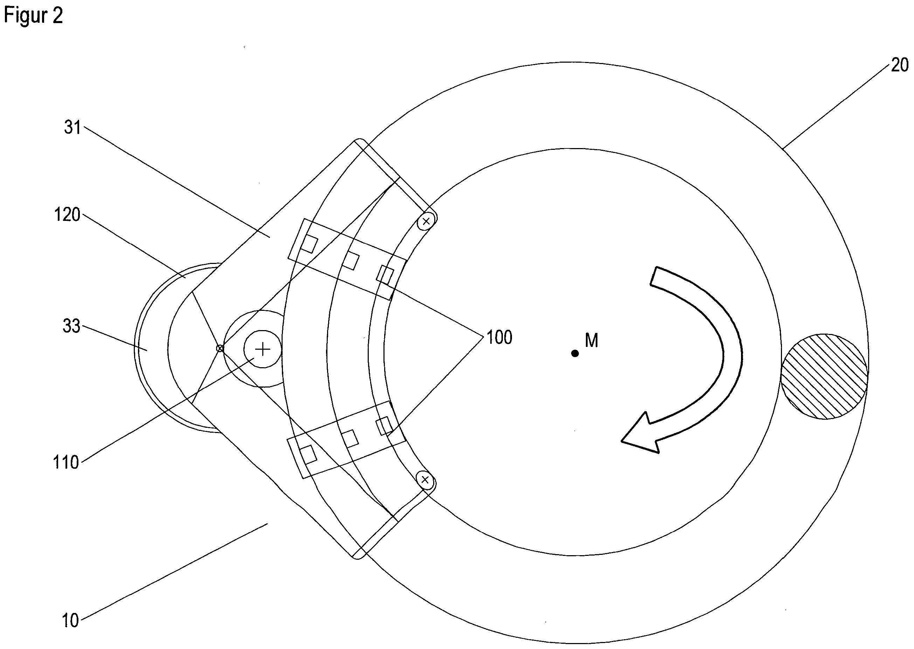

Figur 2 zeigt ein weiteres Ausführungsbeispiel, bei dem zwei voneinander beabstandete ringförmige UV-LEDs 100 vorgesehen sind. Zwischen diesen befindet sich die Rolle 110, die durch einen Motor angetrieben wird. Dessen Stromversorgung 120 kann grundsätzlich über die Achse bzw. das Befestigungselement erfolgen, mittels dessen die Griffanordnung befestigt wird oder auch autark, z.B. mittels einer Batterie oder mittels eines Akkus.

Der Ring kann in seiner Draufsicht kreisrund sein oder auch eine davon abweichende Form aufweisen. Dies gilt für den Querschnitt des Rings entsprechend.

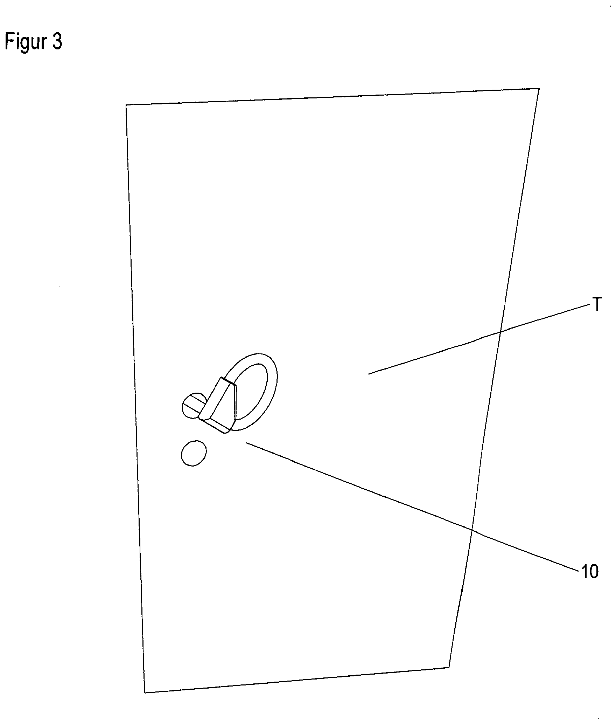

Figur 3 zeigt ein nicht von der vorliegenden Erfindung umfasstes Ausführungsbeipiel der Griffanordnung an einer Zimmertür T.

Die Erfindung umfasst die Fixierung der Griffanordnung an beliebigen Elementen von Blutbehandlungsgeräten, wie Dialysegeräten, wie z.B. an deren Gehäuse oder deren Tür.

## Patentansprüche

1. Blutbehandlungsgerät, mit wenigstens einer Griffanordnung (10) für eine Tür des Geräts, wobei die Griffanordnung wenigstens einen Griff (20) aufweist, der ringförmig ausgebildet ist und dass die Griffanordnung des Weiteren wenigstens eine mit Licht, vorzugsweise mit UV-Licht arbeitende Desinfektionseinheit aufweist, die angeordnet ist, den Griff zu desinfizieren, wobei die Griffanordnung des Weiteren wenigstens eine Antriebseinheit aufweist, die ausgebildet ist, den Griff an der Desinfektionseinheit entlang zu bewegen,
**dadurch gekennzeichnet,**
**dass** der Griff aus einem elektrisch leitenden Material besteht und dass wenigstens ein Berührungssensor vorgesehen ist, der einen Bestandteil der Griffanordnung bildet, anhand eines Schleifkontakts elektrisch leitend mit dem Griff verbunden ist und ausgebildet ist, die Berührung des Griffs zu erfassen und mit zeitlichem Versatz nach einer derartigen Erfassung die Antriebseinheit zu aktivieren.

2. Blutbehandlungsgerät nach Anspruch 1, **dadurch gekennzeichnet, dass** die Antriebseinheit ausgebildet ist, den Griff solange zu bewegen, dass jeder Umfangsabschnitt des Griffes oder der zuvor freiliegende Abschnitt des Griffes zumindest einmal an der der Desinfektionseinheit entlang bewegt wird.

3. Blutbehandlungsgerät nach einem der vorhergehenden Ansprüche, **dadurch gekennzeichnet, dass** die Desinfektionseinheit den Griff teilweise oder vollständig umgreift und vorzugsweise ihrerseits ringförmig ausgebildet ist.

4. Blutbehandlungsgerät nach einem der vorhergehenden Ansprüche, **dadurch gekennzeichnet, dass** eine oder mehrere Führungselemente, insbesondere Führungsrollen (40) vorgesehen sind, die den Griff bei seiner Bewegung führen.

5. Blutbehandlungsgerät nach Anspruch 4, **dadurch gekennzeichnet, dass** wenigstens eines der Führungselemente angetrieben ist und somit die Antriebseinheit oder einen Teil der Antriebseinheit bildet.

6. Blutbehandlungsgerät nach Anspruch 4 oder 5, **dadurch gekennzeichnet, dass** die Antriebseinheit derart ausgebildet ist, dass der Griff in einer Richtung bewegt wird, in der der Griff zunächst an dem oder den Führungselementen entlang bewegt wird und anschließend an der Desinfektionseinheit.

7. Blutbehandlungsgerät nach einem der vorhergehenden Ansprüche, **dadurch gekennzeichnet, dass** die Desinfektionseinheit ausgebildet ist, UV-C oder UV-A Licht abzugeben.

8. Blutbehandlungsgerät nach einem der vorhergehenden Ansprüche, **dadurch gekennzeichnet, dass** der Griff innen hohl ausgeführt ist.

9. Blutbehandlungsgerät nach einem der vorhergehenden Ansprüche, **dadurch gekennzeichnet, dass** der Griff insgesamt oder wenigstens an seiner Oberfläche aus Metall, vorzugsweise aus Edelstahl besteht.

10. Blutbehandlungsgerät nach einem der vorhergehenden Ansprüche, **dadurch gekennzeichnet, dass** der Griff eine Oberfläche oder eine Oberflächenbeschichtung aufweist, die katalytisch oder photokatalytisch wirkende Stoffe aufweist.

11. Blutbehandlungsgerät nach einem der vorhergehenden Ansprüche, **dadurch gekennzeichnet, dass** das Blutbehandlungsgerät ein Dialysegerät ist.

## Claims

1. A blood treatment device having at least one handle arrangement (10) for a door of the device, wherein the handle arrangement has at least one handle (20) that is configured in ring shape; and in that the handle arrangement furthermore has at least one disinfection unit that works with light, preferably with UV light, and that is arranged to disinfect the handle, with the handle arrangement furthermore having at least one drive unit that is configured to move the handle along the disinfection unit.,
**characterized in that**
the handle comprises an electrically conductive material; and **in that** at least one contact sensor that forms a component of the handle arrangement is provided, is electrically conductively connected to the handle by means of a sliding contact, and is configured to detect the contact of the handle and to activate the drive unit with a time offset after such a detection.

2. A blood treatment device in accordance with claim 1, **characterized in that** the drive unit is configured to move the handle for so long that each peripheral section of the handle or the previously exposed section of the handle is moved along the disinfection unit at least once

3. A blood treatment device in accordance with one of the preceding claims, **characterized in that** the disinfection unit engages partly or completely around the handle and is preferably itself configured in ring shape.

4. A blood treatment device in accordance with one of the preceding claims, **characterized in that** one or more guide elements, in particular guide rollers (40), are provided that guide the handle on its movement.

5. A blood treatment device in accordance claim 4, **characterized in that** at least one of the guide elements is driven and thus forms the drive unit or a part of the drive unit.

6. A blood treatment device in accordance with claim 4 or claim 5, **characterized in that** the drive unit is configured such that the handle is moved in a direction in which the handle is first moved along the guide element or elements and subsequently along the disinfection unit.

7. A blood treatment device in accordance with one of the preceding claims, **characterized in that** the disinfection unit is configured to emit UVC light or UVA light.

8. A blood treatment device in accordance with one of the preceding claims, **characterized in that** the handle is hollow at the inside.

9. A blood treatment device in accordance with one of the preceding claims, **characterized in that** the handle comprises metal, preferably stainless steel, overall or at least at its surface.

10. A blood treatment device in accordance with one of the preceding claims, **characterized in that** the handle has a surface or a surface coating that has catalytically or photocatalytically acting substances, in particular titanium dioxide.

11. A blood treatment device in accordance with one of the preceding claims, **characterized in that** the blood treatment device is a dialysis device.

## Revendications

1. Appareil de traitement du sang, comprenant au moins un ensemble poignée (10) pour une porte de l'appareil, l'ensemble poignée comportant au moins une poignée (20), qui est conçue de forme annulaire et en ce que l'ensemble poignée comporte en outre au moins une unité de désinfection fonctionnant à la lumière, de préférence à la lumière UV, qui est agencée pour désinfecter la poignée, l'ensemble poignée comportant en outre au moins une unité d'entraînement, qui est conçue pour déplacer la poignée le long de l'unité de désinfection,
**caractérisé en ce que**
la poignée est constituée d'un matériau électriquement conducteur et **en ce qu'**au moins un capteur de contact est prévu, qui fait partie de l'ensemble poignée, est relié à la poignée de manière électriquement conductrice au moyen d'un contact glissant et est conçu pour détecter le contact avec la poignée et activer l'unité d'entraînement avec un décalage temporel après une détection de ce type.

2. Appareil de traitement du sang selon la revendication 1, **caractérisé en ce que** l'unité d'entraînement est conçue pour déplacer la poignée jusqu'à ce que chaque partie périphérique de la poignée ou la partie précédemment libre de la poignée ait été déplacée au moins une fois le long de l'unité de désinfection.

3. Appareil de traitement du sang selon l'une des revendications précédentes, **caractérisé en ce que** l'unité de désinfection entoure partiellement ou entièrement la poignée et est de préférence pour sa part réalisée de forme annulaire.

4. Appareil de traitement du sang selon l'une des revendications précédentes, **caractérisé en ce qu'**un ou plusieurs éléments de guidage, en particulier des galets de guidage (40) sont prévus, qui guident la poignée dans son déplacement.

5. Appareil de traitement du sang selon la revendication 4, **caractérisé en ce qu'**au moins un des éléments de guidage est entraîné et forme ainsi l'unité d'entraînement ou une partie de l'unité d'entraînement.

6. Appareil de traitement du sang selon la revendication 4 ou 5, **caractérisé en ce que** l'unité d'entraînement est conçue de telle manière que la poignée est déplacée dans une direction, dans laquelle la poignée est d'abord déplacée le long du ou des éléments de guidage puis de l'unité de désinfection.

7. Appareil de traitement du sang selon l'une des revendications précédentes, **caractérisé en ce que** l'unité de désinfection est conçue pour émettre de la lumière UV-C ou UV-A.

8. Appareil de traitement du sang selon l'une des revendications précédentes, **caractérisé en ce que** la poignée est réalisée creuse à l'intérieur.

9. Appareil de traitement du sang selon l'une des revendications précédentes, **caractérisé en ce que** la poignée est constituée, globalement ou au moins sur sa surface, de métal, de préférence d'acier spécial.

10. Appareil de traitement du sang selon l'une des revendications précédentes, **caractérisé en ce que** la poignée comporte une surface ou un revêtement de surface, qui comporte des substances à action catalytique ou photocatalytique.

11. Appareil de traitement du sang selon l'une des revendications précédentes, **caractérisé en ce que** l'appareil de traitement du sang est un appareil de dialyse.
